# FASCICULE DE BREVET EUROPEEN

(11) **EP 0 375 552 B1**
(45) Date de publication et mention de la délivrance du brevet: **14.09.1994**
(21) Numéro de dépôt: 89403586.4
(22) Date de dépôt: 20.12.1989
(51) Int. Cl.: A61B 6/00

(54) **Appareil de radiologie à isocentre mobile dans l'espace**
Röntgengerät mit einem verstellbaren Isozentrum
X-ray apparatus with an adjustable iso centre

(30) Priorité: 22.12.1988 FR 8817000
(43) Date de publication de la demande: 27.06.1990
(73) Titulaire: GENERAL ELECTRIC CGR S.A., F-92130 Issy les Moulineaux (FR)
(72) Inventeur: Chambron, Edmond, F-75116 Paris (FR)
(74) Mandataire: Ballot, Paul Denis Jacques

(56) Documents cités:
- EP-A- 0 165 157
- DE-A- 2 120 344
- FR-A- 2 173 892
- FR-A- 2 588 745
- GB-A- 2 098 440
- US-A- 4 358 856

## Description

L'invention est relative à un appareil d'observation ou d'exploration du corps humain à l'aide de rayons X.

Pour l'examen de certaines parties du corps humain, notamment les vaisseaux sanguins (examen vasculaire), on utilise un faisceau de rayons X dont la direction est variable de manière à pouvoir observer ces parties sous diverses incidences.

Ainsi, pour examiner une zone quasi ponctuelle du corps, le faisceau de rayons X doit tourner - de préférence selon toutes les directions possibles - autour d'un point géométrique, appelé "isocentre", qui est caractéristique de l'appareil d'observation et qui coïncide avec la zone à examiner.

Par ailleurs, il est nécessaire que l'isocentre soit aussi déplaçable dans l'espace selon une courbe de forme quelconque, par exemple pour examiner un vaisseau sanguin lors d'un examen vasculaire.

Un appareil qui assure ces fonctions est appelé statif d'exploration à isocentre mobile. Il comporte une source de rayons X et un détecteur de rayonnement X qui sont en général fixes l'un par rapport à l'autre. Pour la commodité de l'exposé, on se référera dans ce qui suit à un vecteur RX dont la direction est celle du faisceau de rayons X produit par la source vers le détecteur. L'isocentre est aligné avec ce vecteur RX et est situé à un point déterminé entre la source et le détecteur qui dépend du réglage de la source de rayons X.

En plus de la mobilité de l'isocentre, un tel appareil d'exploration doit satisfaire à d'autres exigences qui dépendent du type d'examen qui doit être pratiqué. Ainsi, il faut pouvoir passer d'une position d'exploration à une autre en un temps relativement court sans avoir à arrêter l'exploration. Par ailleurs, les mouvements du praticien, chargé de l'examen, ne doivent pas être entravés et l'encombrement de l'appareil doit être modéré afin que la salle d'examen ne soit pas de dimensions trop importantes. En outre, il est souvent souhaitable de faire varier le grandissement, c'est-à-dire de faire varier la position de la zone à observer sur le vecteur RX.

Un appareil réalisant ces fonctions comprend un support, habituellement un arceau, pour l'ensemble source-détecteur qui permet la rotation du vecteur RX dans un plan. Ce support est solidaire d'un bras qui peut tourner autour d'un axe horizontal distinct de celui autour duquel peut tourner le vecteur RX dans le plan. Ce bras est solidaire d'une colonne verticale le long de laquelle il peut se déplacer. Par ailleurs, l'appareil comporte une table sur laquelle repose le patient à examiner et le plateau de cette table peut se déplacer suivant deux axes situés dans un plan horizontal et un troisième axe vertical. Un appareil de ce type est, par exemple, décrit dans le brevet US 4481656. Un tel appareil est complexe et onéreux.

Un appareil du même type est décrit dans la demande de brevet européen n° 0 165 157 à la différence que le bras est solidaire d'une colonne angulairement réglable dans un plan vertical.

Dans la demande de brevet français publiée n° 2 588 745 il est décrit un appareil de radiologie dans lequel le déplacement de l'isocentre est obtenu par la combinaison d'un déplacement du support, portant l'ensemble source-détecteur, en direction verticale le long de la colonne, d'un déplacement de la table et/ou de la colonne selon une direction horizontale et d'un déplacement de la colonne selon une autre direction horizontale, perpendiculaire à la direction de déplacement de la table.

Un tel appareil permet ainsi trois degrés de liberté pour les mouvements de l'isocentre : un degré de liberté par le déplacement de la table (direction OX), un degré de liberté par le déplacement de la colonne (direction OY) perpendiculairement à la direction OX et un degré de liberté par le déplacement le long de la colonne verticale (direction OZ). Ainsi l'isocentre peut parcourir une forme quelconque dans l'espace.

L'appareil qui vient d'être décrit présente les inconvénients suivants : le déplacement de l'isocentre est obtenu par les mouvements de deux éléments séparés : le statif qui porte l'ensemble source-détecteur et la table qui porte le patient et il n'est pas aisé de combiner et coordonner leurs mouvements, surtout lorsque le praticien en effectue lui-même la commande.

Le praticien n'a pas facilement accès au patient sur la table car ses mouvements sont entravés par la présence du statif.

Un but de la présente invention est donc de réaliser un appareil de radiologie dans lequel le mouvement de l'isocentre est obtenu essentiellement par le déplacement du statif.

Un autre but de la présente invention est de réaliser un appareil de radiologie dans lequel le statif est prévu pour dégager la table de support du patient et ainsi permettre un accès sans entraves des deux côtés de la table.

L'invention est relative à un appareil de radiologie à isocentre mobile dans l'espace qui comprend une table disposée parallèlement par rapport à un axe longitudinal et horizontal X'X et à un axe transversal et horizontal Y'Y et un statif, ledit statif comportant un ensemble source-détecteur porté par un arceau qui coulisse dans une gaine, la gaine étant montée pivotante autour d'un premier axe horizontal et porté par un chariot, lui-même monté coulissant sur une colonne dont l'extrémité inférieure pivote autour d'un deuxième axe horizontal parallèle au premier axe horizontal, ce deuxième axe horizontal étant monté sur une embase dont son extrémité opposée à celle de l'axe de la colonne est elle-même montée pivotante dans un plan horizontal autour d'un axe vertical, de manière que le mouvement de l'isocentre soit obtenu essentiellement par le déplacement du statif.

Le pivotement de la colonne autour dudit deuxième axe horizontal parallèle au premier axe horizontal permet de déplacer l'ensemble source-détecteur dans un plan vertical, tandis que le déplacement pivotant de l'embase permet de la déplacer autour de la table. Le déplacement du chariot sur la colonne permet le déplacement vertical de l'ensemble source-détecteur.

La rotation de la gaine autour du premier axe horizontal permet de choisir l'angle d'incidence des rayons X par rapport à un axe horizontal et transversal au premier axe horizontal tandis que la rotation de l'arceau permet de choisir l'incidence des rayons X par rapport au premier axe horizontal.

Le déplacement de l'embase permet également de dégager la tête du patient en dehors de l'examen proprement dit. Selon un autre perfectionnement particulièrement avantageux de l'invention, l'embase est montée pivotante autour d'un axe vertical qui est confondu avec l'axe de rayonnement de la source lorsque l'arceau et la colonne sont disposés dans le même plan vertical.

Ceci permet d'obtenir des mouvements d'exploration qui sont symétriques de part et d'autre du patient et donc de dégager un côté ou l'autre du patient pour en permettre l'accès au praticien.

D'autres caractéristiques et avantages de la présente invention apparaîtront à la lecture de la description suivante d'un exemple particulier de réalisation, ladite description étant faite en relation avec les dessins joints dans lesquels :
- la figure 1 est une vue en perspective cavalière d'un exemple de réalisation d'un statif de radiologie selon l'invention, et
- la figure 2 est une vue en perspective cavalière d'un statif de radiologie semblable à celui de la figure 1 mais présentant une disposition particulière de l'ensemble source-détecteur par rapport à l'arceau de support.

L'appareil de radiologie selon l'invention comprend un statif de radiologie 10 et une table 11 de support d'un patient. Pour des raisons de clarté du dessin, la table 11 a été représentée uniquement sous la forme d'un plan horizontal mais on comprendra qu'elle est supportée par un pied qui comporte de manière connue des dispositifs de déplacement suivant trois axes orthogonaux X'X, Y'Y et Z'Z.

Le statif 10 comprend une source 13 de rayons X et au moins un détecteur 14 de rayonnement X montés sur les deux extrémités diamétralement opposées d'un arceau 15 en forme d'arc-de-cercle. La source 13 est associée à des moyens de commande, non représentés, tandis que le ou les détecteurs de rayonnement X sont associés à des moyens d'imagerie, non représentés, qui permettent la photographie et/ou la reproduction d'une image sur un écran de visualisation tel que celui d'un tube à rayons cathodiques et, éventuellement, la mémorisation dans un ordinateur.

L'arceau 15 est porté par une gaine 16 dans laquelle il peut coulisser sous la commande d'un dispositif de déplacement, connu par ailleurs, de manière à obtenir une rotation autour d'un axe passant par le centre de l'arceau. La gaine 16 est supportée par un chariot 17 et tourne autour d'un axe 18 solidaire du chariot 17. Le chariot 17 est monté coulissant sur une colonne 19 dont l'extrémité inférieure pivote autour d'un axe 20 porté par une embase 21. La double flèche 25 indique les sens de déplacement du chariot 17 sur la colonne 19. De même, la double flèche 26 indique les deux sens de rotation de la colonne 19.

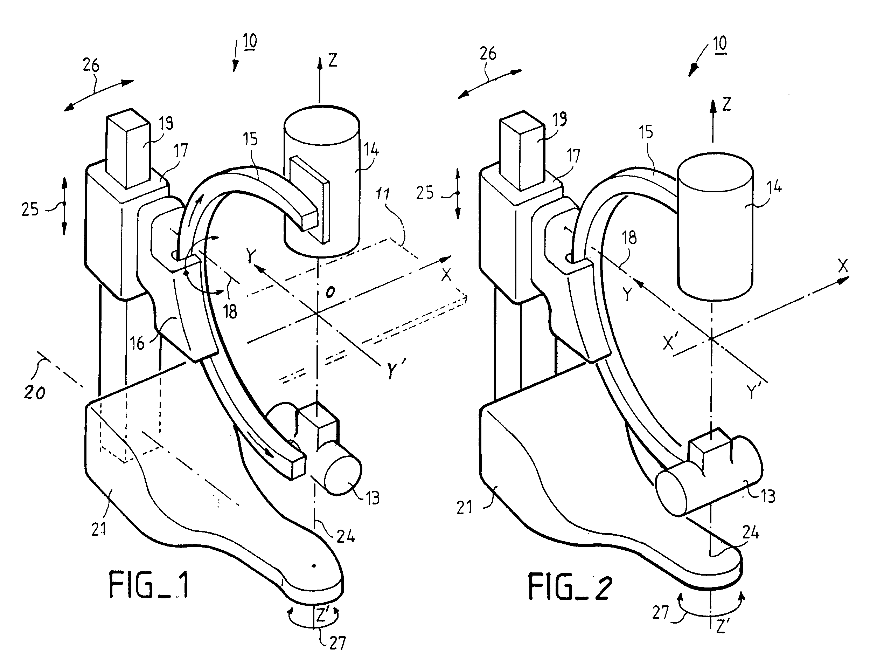

Dans les exemples de réalisation des figures 1 et 2, l'embase 21 pivote autour d'un axe vertical 24.

Sur les différentes figures, les moyens qui permettent de réaliser les différents mouvements n'ont pas été représentés car ils sont connus par ailleurs ou sont à la portée de l'homme de métier sans faire oeuvre d'invention.

Les différents axes de déplacement et de rotation ont, de préférence, les orientations suivantes dans l'espace : les axes 18, 20 sont parallèles à un premier axe horizontal et l'axe 24 (figures 1 et 2) est vertical comme déjà indiqué ci-dessus. En outre, cet axe 24 est confondu avec le vecteur RX lorsque l'arceau 15, l'ensemble source-détecteur et la colonne 19 sont disposés verticalement.

Avec l'appareil de radiologie représenté sur les figures, tous les mouvements, qu'ils soient de déplacement rectiligne ou de rotation, sont indépendants les uns des autres. C'est ainsi que :
- la colonne 19 pivote autour d'un axe 20 parallèle au premier axe horizontal,
- le chariot 17 se déplace sur la colonne 19,
- la gaine 16 pivote autour d'un axe 18 parallèle au premier axe horizontal,
- enfin, l'arceau 15 coulisse dans la gaine 16 en pivotant autour d'un point,
- l'embase 21 pivote autour de l'axe 24.

Ces différents mouvements indépendants peuvent être contrôlés par un système de commande électronique approprié et assisté par ordinateur de manière que l'isocentre de l'appareil décrive n'importe quelle courbe dans le volume d'examen où est disposé le patient. On peut ainsi réaliser l'examen vasculaire d'un membre du patient ou d'un vaisseau sanguin du corps proprement dit.

C'est ainsi que la combinaison des mouvements de rotation de la colonne 19, le coulissement du chariot 17 et la rotation de la gaine 16 permettent de déplacer l'isocentre sur un axe horizontal et transversal au premier axe horizontal.

Dans les appareils de radiologie représentés sur les figures 1 et 2, l'embase 21 pivote autour de l'axe vertical 24 dont la position a été définie ci-dessus, et qui permet, selon un sens indiqué par la double flèche 27, de déplacer l'embase 21 dans deux directions parallèles l'une au premier axe horizontal et l'autre à un axe horizontal et transversal au premier axe horizontal. En outre, le pivotement permet d'effectuer le déplacement de l'ensemble source-détecteur de part et d'autre de la table 11 en fonction du type d'examen. Ainsi, l'examen peut être réalisé dans les conditions optimales pour le praticien et les mesures radiologiques. Enfin, le statif peut être éloigné suffisamment de la tête du patient pour en permettre l'accès au praticien.

Les appareils de radiologie des figures 1 et 2 sont identiques à l'exception de la position de l'ensemble source-détecteur par rapport à l'arceau. Sur la figure 2, l'ensemble source-détecteur est dans le plan de l'arceau 15, ce qui est habituel en radiologie; par contre, sur la figure 1 l'ensemble source-détecteur est disposé dans un plan parallèle à l'arceau 15. Cette deuxième disposition présente les avantages suivants :
- la longueur de coulissement de l'arceau est plus grande car les extrémités de l'arceau dans son plan sont dégagées;
- la source et le détecteur peuvent être plus facilement écartés ou rapprochés l'un de l'autre car leur débattement n'est pas gêné par l'arceau;
L'appareil de radiologie selon l'invention qui vient d'être décrit présente de nombreux avantages tels que :
- l'exploration tête-pieds du patient sans bouger ce dernier,
- le dégagement de la tête du patient pour en permettre l'accès par le praticien,
- le déplacement de la partie mécanique au sol vers un secteur de la salle non utilisé par le praticien,
- la réduction des masses d'inertie à déplacer.

## Revendications

1. Un appareil de radiologie à isocentre mobile dans l'espace qui comprend une table (11) disposée parallèlement par rapport à un axe longitudinal et horizontal X'X et un axe transversal et horizontal Y'Y et un statif (10), ledit statif (10) comportant un ensemble source-détecteur (13, 14) porté par un arceau (15) qui coulisse dans une gaine (16), la gaine étant montée pivotante autour d'un premier axe horizontal (18) et porté par un chariot (17), lui-même monté coulissant sur une colonne (19) dont l'extrémité inférieure pivote autour d'un deuxième axe horizontal (20) parallèle au premier axe horizontal (18), caractérisé en ce que ce deuxième axe horizontal (20) est monté sur une embase (21) dont son extrémité opposée à celle de l'axe (20) de la colonne (19) est elle-même montée pivotante dans un plan horizontal autour d'un axe vertical (24), de manière que le mouvement de l'isocentre soit obtenu essentiellement par le déplacement du statif.

2. Appareil de radiologie selon la revendication 1, caractérisé en ce que ledit axe vertical (24) est confondu avec l'axe de rayonnement de la source (13) de rayons X lorsque l'arceau (15) et la colonne (19) sont disposés dans le même plan vertical.

3. Appareil de radiologie selon l'une quelconque des revendications précédentes, caractérisé en ce que la source (13) et le détecteur (14) sont disposés aux extrémités de l'arceau (15) de manière que l'axe du rayonnement X soit situé dans le plan de l'arceau (15).

4. Appareil de radiologie selon l'une des revendications 1 ou 2, caractérisé en ce que la source (13) et le détecteur (14) sont disposés latéralement aux extrémités de l'arceau (15) de manière que l'axe du rayonnement X soit situé dans un plan parallèle à celui de l'arceau (15).

## Claims

1. A radiological device with an isocentre that may be moved in space having a table (11) located parallel in relation to a longitudinal, horizontal axis X'X and a transversal, horizontal axis Y'Y and a stand (10), the stand (10) having a source-detector (13, 14) assembly mounted on an arcuate member (15) which slides in a support (16), the support being mounted so that it pivots around a first horizontal axle (18) and mounted on a carriage (17), itself slidably mounted on a column (19), the lower end of which pivots around a second horizontal axle (20) parallel to the first horizontal axle (18), characterised in that this second horizontal axle (20) is mounted on a base (21) whose end opposite to that of the axle (20) of the column (19) is itself mounted so as to pivot in a horizontal plane around a vertical axle (24) so that the isocentre is moved essentially by moving the stand.

2. A radiological device in accordance with claim 1, characterised in that the vertical axle (24) merges with the axis of radiation from the X-ray source (13) when the arcuate member (15) and the column (19) are located in the same vertical plane.

3. A radiological device in accordance with one of the previous claims, characterised in that the source (13) and the detector (14) are located at the ends of the arcuate member (15) so that the axis of the X-ray is located in the plane of the arcuate member (15).

4. A radiological device in accordance with one of claims 1 or 2, characterised in that the source (13) and the detector (14) are arranged laterally at the ends of the arcuate member (15) so that the axis of the X-rays is located in a plane parallel to that of the arcuate member (15).

## Patentansprüche

1. Röntgengerät mit im Raum verstellbarem Isozentrum, mit einem parallel bezüglich einer in Längsrichtung horizontal verlaufenden Achse X'X und einer quer und horizontal verlaufenden Achse Y'Y angeordneten Tisch (11) und einem Stativ (10), wobei das Stativ (10) eine Quelle-Detektor-Baueinheit (13, 14) enthält, die von einem Bügel (15) getragen wird, der in einem Mantel (16) gleitet, wobei der Mantel um eine erste horizontale Achse (18) schwenkbar gelagert ist und von einem Schlitten (17) getragen ist, der seinerseits auf einer Säule (19) verschiebbar angebracht ist, deren unteres Ende um eine zweite horizontale Achse (20) parallel zur ersten horizontalen Achse (18) schwenkbar ist, dadurch gekennzeichnet, daß die zweite horizontale Achse (20) an einem Sockel (21) angebracht ist, dessen dem Ende der Achse (20) der Säule (19) gegenüberliegendes Ende seinerseits in einer horizontalen Ebene um eine vertikale Achse (24) schwenkbar angebracht ist, so daß die Bewegung des Isozentrums im wesentlichen durch die Verschiebung des Stativs erhalten wird.

2. Röntgengerät nach Anspruch 1, dadurch gekennzeichnet, daß die vertikale Achse (24) mit der Achse der Röntgenstrahlenquelle (13) zusammenfällt, wenn der Bügel (15) und die Säule (19) in der gleichen Vertikalebene angeordnet sind.

3. Röntgengerät nach einem der vorhergehenden Ansprüche, dadurch gekennzeichnet, daß die Quelle (13) und der Detektor (14) an den Enden des Bügels (15) so angebracht sind, daß die Achse der Röntgenstrahlung in der Ebene des Bügels (15) liegt.

4. Röntgengerät nach einem der Ansprüche 1 oder 2, dadurch gekennzeichnet, daß die Quelle (13) und der Detektor (14) seitlich der Enden des Bügels (15) so angebracht sind, daß die Achse der Röntgenstrahlung in einer Ebene liegt, die parallel zu der des Bügels (15) verläuft.
